# EUROPEAN PATENT SPECIFICATION

(11) **EP 0 474 666 B1**
(45) Date of publication and mention of the grant of the patent: **14.09.1994**
(21) Application number: 90907573.1
(22) Date of filing: 15.05.1990
(51) Int. Cl.: C07C 62/38, C07J 71/00, C07D 303/38

(54) **OXIDATIVE PREPARATION OF A-NOR-17 BETA-CARBOXY-3,5-SECOANDROSTAN-5-ONE-3 OIC ACID**
OXIDATIVE HERSTELLUNG VON A-NOR-17-BETA-CARBOXY-3,5-SECO-ANDROSTAN-5-ON-3-SÄURE
PREPARATION PAR OXYDATION DE L'ACIDE A-NOR-17 BETA-CARBOXY-3,5-SECOANDROSTAN-5-ONE-3 OIQUE

(30) Priority: 30.05.1989 US 358800; 09.01.1990 US 462417
(43) Date of publication of application: 18.03.1992
(73) Proprietor: THE UPJOHN COMPANY, Kalamazoo, Michigan 49001 (US)
(72) Inventor: HERRINTON, Paul, Matthew, Kalamazoo, MI 49002 (US)
(74) Representative: Perry, Robert Edward
(86) International application number: US9002622
(87) International publication number: WO9015045

(56) References cited:
- FR-A- 1 510 055
- US-A- 2 727 905
- US-A- 4 220 775
- Journal of Organic Chemistry, vol. 34, no. 1, Jan. 1969, Washington, DC, US; D.M. PIATAK et al.: "Oxidation of steroidal ketones. VII. Cleavage of steroidal conjugated ketones with ruthenium tetroxide", p. 112-116
- Journal of the American Chemical Society, vol. 78, No. 7, 5 April 1959, Washington, DC, US; E.P. OLIVETO et al.: "11-Oxygenated steroids. XIV. New syntheses of corticosterone", p. 1414-1416

## Description

The present invention relates to a process for the preparation of 3,5-secoandrost-5-one-3,17β-dioic acids.

### Background of the Invention

The oxidation of the steroid A-ring by ozone (O₃) is known; see J.O.C. 23:1787 (1958), US-A-3285918 and EP-A-0277002.

The oxidation of the steroid A-ring by MnO₄⁻¹/IO₄⁻¹ is known; see J. Med. Chem. 27:1690 (1984) and EP-A-0277002.

The oxidation of a steroidal C₁₇ enone side-chain to the corresponding 17β-COOH by ozone has been reported; see J.A.C.S. 78:1414 (1956). The cleavage of 1,3-dicarbonyls is known; see J.C.S. Chem. Comm. 1055 (1968).

The known methods of producing 17β-carboxylic-3,5-secoandrostanes require that the particular C₁₇ side-chain desired in the secosteroid (II) be present in the starting material to be oxidised or that the starting material includes a group which, following oxidation of the steroid A-ring, can readily be converted to the desired C₁₇ side-chain; see, for example, EP-A-0277002.

### Summary of the Invention

According to a first aspect of the present invention, a 3,5-secoandrost-5-one-3,17β-dioic acid (II) is prepared by contacting a 21-unsaturated progesterone (I) with an oxidising agent such as ozone. Formulae I and II are given and defined in claim 1.

The process of the present invention differs from known methods because the one-step process involves oxidation of both ends of the steroid molecule in a simultaneous reaction. The use of glyoxylates as 1,3-dicarbonyls in an oxidation reaction is novel. The oxidation of an alkoxyoxalkyl group to give a carboxylic acid has not previously been reported.

Further aspects of the present invention are the compounds of formulae I-C and II-C defined in claims 16 and 17. In these compounds, the C-ring is an 9β,11β-epoxide.

### Detailed Description of the Invention

The 21-unsaturated progesterones (I) are known compounds; see US-A-2727905, US-A-4325878 and J.A.C.S. 82:1709 (1960). Alternatively, they can readily be prepared from known substituted progesterones by methods known to those skilled in the art; see, for example, US-A-2727905 and Gazz. Chim. Ital. 84:312 (1954). In Chart A and the claims, the C₁₇ side-chain is set forth as -CO-CH=C(R₂₂₋₁)(R₂₂₋₂); when R₂₂₋₁ is -OH, this side-chain, as is known to those skilled in the art, is equivalent to -CO-CH₂-CO-R₂₂₋₂. It is preferred that R₂₂₋₁ is -OH and R₂₂₋₂ is -CO-O-R₂₂₋₁₁. It is preferred that R₂₂₋₁₁ is C₁-C₄ alkyl, more preferably C₁-C₂ alkyl.

There are two different ways to practise the process of the present invention. The first method involves contacting the 21-unsaturated progesterones (I) with, say, O₃, followed by aqueous hydroxide or a means for generating hydroxide, and then neutralising the hydroxide with acid. The 21-unsaturated progesterones (I) need to be dissolved in an appropriate solvent or mixture thereof. Suitable solvents include methylene chloride, acetic acid, chloroform, methanol, ethanol, propanol, isopropanol, butanol, ethyl acetate, DMSO, acetone, dioxane, water and mixtures thereof, depending on the particular 21-unsaturated progesterone (I). The preferred solvent is a methylene chloride/acetic acid mixture. The ozonolysis is operable in a temperature range of about 0 to about -100°, preferably about -20 to about -80°, more preferably about -78°. While it is operable to add aqueous hydroxide to the reaction mixture, it is preferred that the aqueous hydroxide be added in two steps. First, just water and then solid hydroxide. Means for generating hydroxide include, bicarbonate, carbonate or an amine. Amines, even those that are not very water soluble, generate hydroxide. They react with water, abstracting a proton from the water molecule generating an amine cation and hydroxide (anion). The reaction of the amines (A) and water can be visualized as A + H₂O ---→ A-H⁺ + OH⁻. Operable amines include, for example, diethylamine, triethylamine, pyridine, aniline, piperazine, pyrrolidine, piperidine, morpholine, 1-methylpiperidine, α-naphthylamine, imidazole and substituted imidazoles, 1,2,4-triazole and substituted 1,2,4-triazoles, benzimidazole and substituted benzimidazoles, and equivalents thereof. For example, if the amine was triethylamine or pyridine, the cation generated would be (CH₃CH₂)₃NH⁺ and pyridinium respectively along with hydroxide. It is preferred the hydroxide be sodium or potassium hydroxide. It is desirable to warm the reaction mixture either before or after the addition of the aqueous base to prevent the reaction mixture from freezing as is known to those skilled in the art. It is preferred to warm the reaction mixture to about 0°.

When the 21-unsaturated progesterone (I) is contacted with O₃ the contacting is effectuated by bubbling/sparging O₃ gas thru a solution of the 21-unsaturated progesterone (I). While the reaction will proceed with less than 2 equivalents of O₃, it is preferred that at least 2 equivalents of O₃ be used since that amount is required for the reaction to go to completition. Any large excess is not harmful, just wasteful. As a practical matter the reaction mixture is contacted with somewhat greater than 2 equivalents. It is preferred to remove the excess O₃ from the reaction mixture prior to contacting with aqueous hydroxide. The O₃ is removed by sparging with oxygen, and then the oxygen is removed by sparging with an inert gas such as nitrogen. If the excess O₃ is removed, prior to contacting with the aqueous hydroxide, then it is preferred to contact the reaction mixture with H₂O₂ prior to contacting the reaction mixture with the aqueous hydroxide. Following the addition of the aqueous hydroxide, the excess hydroxide is neutralized by the addition of an acid. Since the only purpose of the acid is to neutralize the hydroxide virtually any moderately strong or strong acid is operable. Preferred acids are hydrochloric, sulfuric, phosphoric, nitric, acetic, perchloric, trifluoroacetic, citric, succinic, maleic, benzoic and p-TSA, more preferred are sulfuric and hydrochloric.

Using the second method, the 21-unsaturated progesterones (I) are dissolved in an appropriate solvent, as discussed above, and contacted with an oxidizing agent. The first type of oxidizing agent consists of an oxidant or means of generating the oxidant. The oxidant is selected from the group consisting of RuO₄, persulfate, pervanadate, pertungstate or MnO₄⁻¹. This oxidant can be used stoichiometrically, or preferably, it is used catalytically with a means for producing the oxidant, a reoxidant. Means for producing the oxidant include is selected from the group consisting of NaOCl, NaClO₄, IO₄⁻¹ and Ca(OCl)₂. Even catalytic amounts of the oxidant are operable provided 4 or more equivalents of the means for generating the oxidant (reoxidant) are present as is more fully discussed below.

Alternatively, the oxidizing agent is a two stage oxidant. With the two stage oxidants, it is believed the first oxidant oxidizes the 21-unsaturated progesterone (I) to a glycol and the second oxidant cleaves the glycol. The two stage oxidants include, for example, OsO₄/Pb(OAc)₄, KMnO₄/Pb(OAc)₄, OsO₄/IO₄⁻¹. The preferred two stage oxidants include osmium tetroxide followed by periodate or lead tetraacetate, and permanganate followed by periodate.

The oxidation reaction using the oxidizing agent is operable in a temperature range of about -100 to about 65°, preferably about -80 to about 40°. The reaction requires a total of 4 equivalents of the oxidizing agent. If catalytic amounts of the oxidant are used, then 4 equivalents of the reoxidant (such as IO₄⁻¹) are required. Alternatively, if 2 equivalents of the oxidant (for example, OsO₄) are used then 2 equivalents of the reoxidant (for example, IO₄⁻¹) are required. The reaction will proceed with less than 4 equivalents of the oxidizing agent but not to completion. It is therefore preferred that at least 4 equivalents of the oxidizing agent or a means for generating be used.

It is preferred that following the contacting of the 22-saturated progesterone (I) with the oxidizing agent, that aqueous hydroxide be added. While it is operable to add aqueous hydroxide to the reaction mixture, it is preferred that the aqueous hydroxide be added in two steps. First, just water and then solid hydroxide. Means for generating hydroxide include, bicarbonate, carbonate or an amine. It is preferred the base be sodium or potassium hydroxide. Following the addition of the aqueous hydroxide, the excess hydroxide is neutralized by the addition of an acid. Virtually any moderately strong or strong acid is operable. Preferred acids are hydrochloric, sulfuric, phosphoric, nitric, acetic, perchloric, trifluoroacetic, citric, succinic, maleic, benzoic and p-TSA. It is desirable to warm the reaction mixture either before or after the addition of the aqueous base to prevent the reaction mixture from freezing as is known to those skilled in the art. It is preferred to warm the reaction mixture to about 0°.

The final product, the seco-steroid (II) is extracted into a variety of organic solvents. Preferred is ethyl acetate or methylene chloride. If methylene chloride is used the seco-steroid (II) can be converted to the 4-aza steroids directly. If it is desired to crystallize the seco-steroid (II), it is preferred to use ethyl acetate for isolation.

The seco-steroid (II) is useful in producing Δ⁵-4-aza-17-carbonyl steroids (III) which are transformed to 5α-4-aza amides (IV), useful pharmaceuticals, all by means well known to those skilled in the art, see, for example, European Patent Application 88300697.5 published 3 August 1988 as European Publication Number 277,002 A2, US Patents 4,325,878 and 4,377,584, J. Steroid Biochem., 19, 385 (1983), J. Med. Chem., 27, 1690 (1984). With the Δ⁵-4-aza-17-carbonyl steroids (III) R₄ is -H or C₁-C₄ and R₂₀ is -OH (acid), -OR₂₀₋₁ (esters) where R₂₀₋₁ is C₁-C₄ alkyl or -φ, and -NR₂₀₋₂R₂₀₋₃ (amides) where R₂₀₋₂ and R₂₀₋₃ are the same or different and are -H or C₁-C₃ alkyl.

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)H₂. When chemical formulas are drawn in a linear fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom. Also, for any molecule with an established system of carbon atom numbering, such as steroids, these carbon atoms are designated as Cᵢ, where "i" is the integer corresponding to the carbon atom number. For example, C₆ represents the 6 position or carbon atom number in the steroid nucleus as traditionally designated by those skilled in the art of steroid chemistry. Likewise the term "R₆" represents a variable substituent (either monovalent or bivalent) at the C₆ position.

Chemical formulas or portions thereof drawn in a linear fashion represent atoms in a linear chain. The symbol "-" in general represents a bond between two atoms in the chain. Thus CH₃-O-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (*) are bonded to each other resulting in the formation of a ring. Likewise, the cyclic molecular fragment, 4-(ethyl)-1-piperazinyl can be represented by -N*-(CH₂)₂-N(C₂H₅)-CH₂-C*H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In chemical structural formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as-C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, the two monovalent variable substituents are α-R₆₋₁:β-R₆₋₂, .... α-R₆₋₉:β-R₆₋₁₀, etc, giving -C(α-R₆₋₁)(β-R₆₋₂)-, .... -C(α-R₆₋₉) (β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CO-O-CH₂-CH₂-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-O-CO- where n is zero, one or two. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6. carbon atoms, the two definitions differ since the former definition allows either the alkoxy or alkyl portion alone to contain 4 or 5 carbon atoms while the latter definition limits either of these groups to 3 carbon atoms.

### II. DEFINITIONS

All temperatures are in degrees Centigrade.

O₃ refers to ozone.

Hydroxide refers to OH⁻¹ and when the term is used in the specification and claims it includes a means for generating hydroxide.

-φ refers to phenyl.

DMSO refers to dimethylsulfoxide.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, solubility, patient acceptance and bioavailability.

When solvent pairs are used, the ratios of solvents used are volume/volume (v/v).

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### PREPARATION 1 21-Benzylidenepregn-4-ene-3,20-dione (I)

Progesterone 3-methyl enol ether is slurried in methanol (13 ml) and benzaldehyde (3.4 ml) is added. Sodium methoxide (25% wt/wt in methanol, 13 ml) is added, the mixture heated under reflux for 2 hr, and then cooled to 20-25°. The pH is adjusted to 1 by the addition of hydrochloric acid (6N, 15 ml) and the mixture stirred at 30° for 2 hr. Water (30 ml) is added and the mixture filtered to give the title compound.

### EXAMPLE 1 3,5-Secoandrost-5-one-3,17β-dioic acid (II)

21-oxalylpregn-4-ene-3,20-dione methyl ester [I, Gazz. Chim. Ital., 84, 312 (1954), 20 g] is dissolved in methylene chloride (100 ml). Acetic acid (3.4 g) is added and the mixture is cooled to < -70°. Ozone is sparged through the mixture until excess ozone is present as indicated by a green color. Oxygen is then sparged until the excess ozone is removed and then nitrogen is sparged until the oxygen is removed. The mixture is then warmed to 0° and water (24 ml) is added. After the mixture warms to 20° hydrogen peroxide (30%, 5 ml) is added and the mixture stirred for 1 hr. Sodium hydroxide (10%, 50 ml) is added. The pH is adjusted to 2 by the addition of concentrated sulfuric acid (16 ml) and methylene chloride is added to dissolve the product. The organic mixture is washed with water (2 x 50 ml) and concentrated (about 40 ml). The solvent is exchanged to ethyl acetate (40 ml), the mixture cooled to 0° and then hepatane (100 ml) is added to crystallize the title compound, mp 197.3-202.6°.

### EXAMPLE 2 3,5-Secoandrost-5-one-3,17β-dioic acid (II)

21-Benzylidenepregn-4-ene-3,20-dione (I, PREPARATION 1, 20 g) is dissolved in methylene chloride (80 ml) and methanol (20 ml). The solution is cooled to -60° and ozone added until an excess is present as indicated by a light blue color. Water (10 ml) is added and the solution warmed to 20-25°. The resulting solution is extracted with aqueous sodium hydroxide (5%, 100 ml). The aqueous solution is acidified to pH 2 by addition of hydrochloric acid (50%) and extracted with ethyl acetate (100 ml). The organic phase is dried over sodium sulfate and concentrated to 40 ml by distillation under reduced pressure. The mixture is cooled to 0° for one hr and the product collected by filtration to give the title compound, mp 203-205°.

### EXAMPLE 3 3,5-Secoandrost-5-one-3,17β-dioic acid (II)

A solution of 21-oxalylpregn-4-ene-3,20-dione methyl ester (I, 7.88 g) in methylene chloride (20 ml) and methanol (5 ml) is cooled to -40° and ozone added until an excess is present as indicated by the presence of a light blue color. Aqueous potassium carbonate (10%, 20 ml) is added and the mixture warmed to 20-25°. The phases are separated and the aqueous phase acidified to pH 2 by addition of aqueous sulfuric acid (10%). The mixture is extracted with ethyl acetate (50 ml). The organic phase is concentrated to dryness to provide the title compound, mp 192-208°.

### EXAMPLE 4 3,5-Secoandrost-5-one-3,17β-dioic acid (II)

A solution of potassium carbonate (5.5 g in 25 ml of water) is added to a solution of 21-oxalylpregn-4-ene-3,20-dione methyl ester (I, 3.9 g) in t-butanol (100 ml). A solution of sodium metaperiodate (3.0 g in 20 ml of water) is prepared and 2 ml of this solution is added in one portion to the steroid solution. A solution of potassium permanganate (0.6 g in 30 ml of water) is prepared and 10 ml is added to the steroid solution. The reaction mixture is kept at 40° during the reaction. The remaining periodate and permanganate solutions are added dropwise over 30 min. After stirring at 40° for 3 hr after the additions are complete the mixture is filtered through celite and enough sodium bisulfite added to discharge the pink color. The mixture is then concentrated to half volume under reduced pressure and extracted with ethyl acetate (50 ml). The organic phase is dried and concentrated to 10 ml volume and cooled. The product crystals are collected by filtration to give the title compound, 193-201°.

### EXAMPLE 5 3,5-Secoandrost-5-one-3,17β-dioic acid (II)

A solution of ruthenium tetroxide (RuO₄) is prepared by suspending ruthenium dioxide (RuO₂, 0.169 g) in acetone (10 ml) and adding a solution of sodium metaperiodate (2.5 g in 10 ml of water). A solution of 21-oxalylpregn-4-ene-3,20-dione methyl ester (I, 1.0 g) in acetone (10 ml) is added dropwise to the ruthenium tetroxide solution. As the solution changes color from yellow to black, a solution of sodium metaperiodate (2.5 g in 10 ml of water) is added to maintain the yellow color. After 4 hours the excess ruthenium tetroxide is is reacted by the addition of isopropanol. The mixture is filtered through celite and concentrated under reduced pressure to remove the acetone. The product is then extracted with ethyl acetate (20 ml). This solution is then dried over sodium sulfate and concentrated to dryness to give the title compound, mp 189-203°.

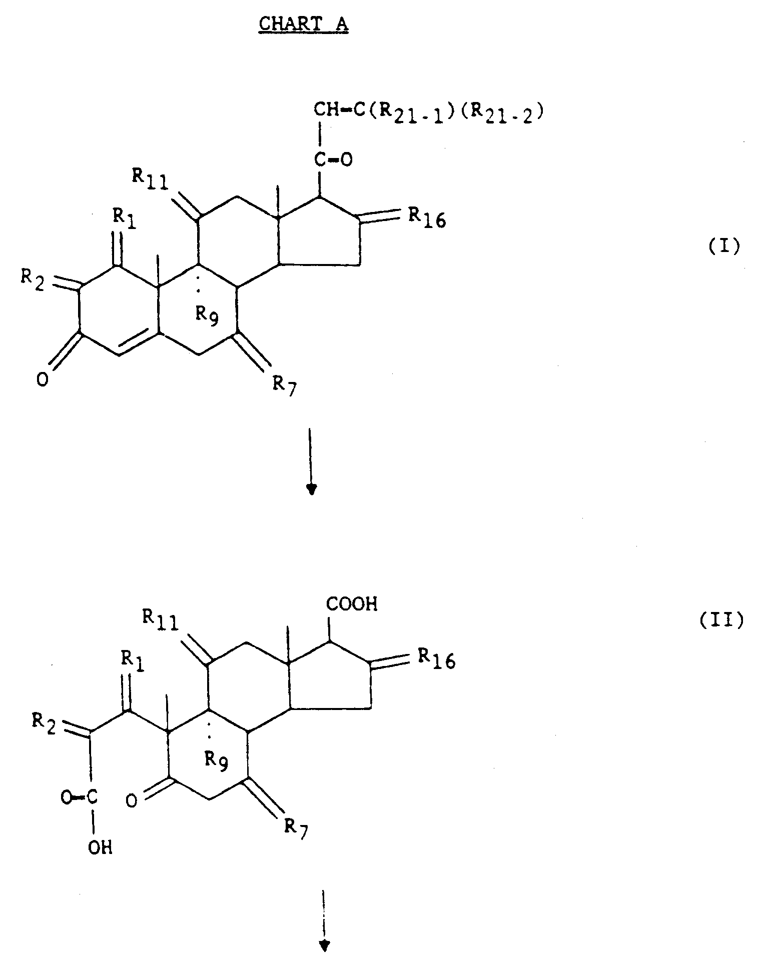

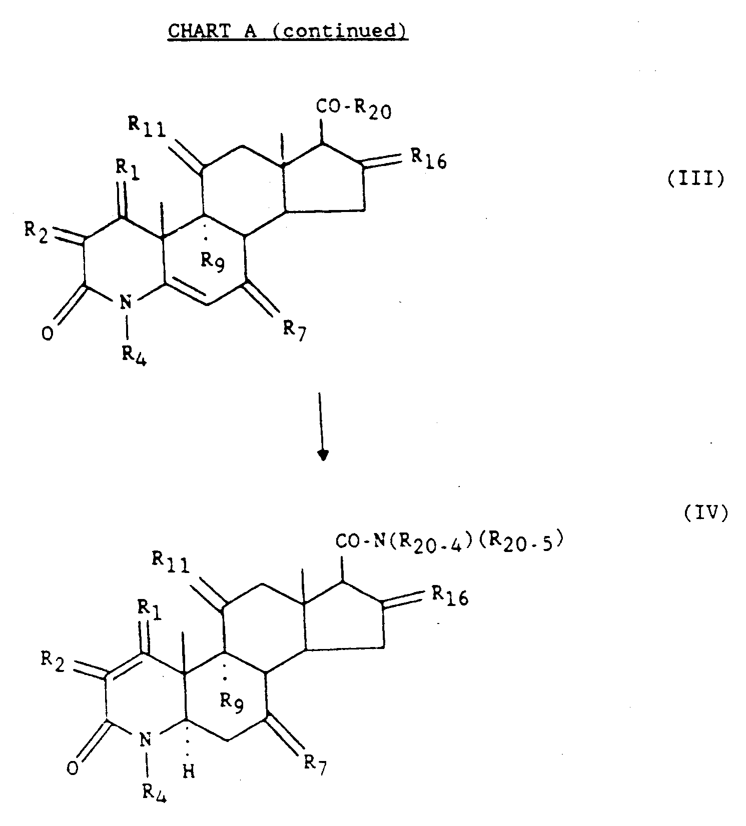

## Claims

1. A process for the production of a 3,5-secoandrostan-5-one-3,17β-dioic acid of formula (II) 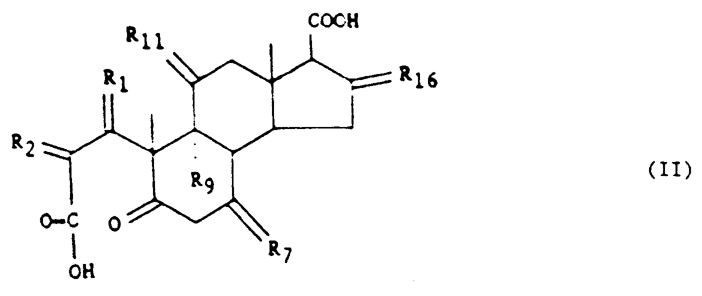 where (A-I) R₁ is α-R₁₋₁:β-R₁₋₂ where one of R₁₋₁ and R₁₋₂ is -H and the other of R₁₋₁ and R₁₋₂ is -H, -F, -Cl, -Br, -I, -OR₁₋₃ where R₁₋₃ is -H or C₁-C₆ alkyl, -SR₁₋₃ where R₁₋₃ is as defined above, R₂ is -H:-H;
(A-II) R₁ is -H:-H, R₂ is α-R₂₋₁:β-R₂₋₂ where one of R₂₋₁ and R₂₋₂ is -H and the other of R₂₋₁ and R₂₋₂ is -H, -F, -Cl, -Br, -I, -OR₂₋₃ where R₂₋₃ is -H or C₁-C₆ alkyl, -SR₂₋₃ where R₂₋₃ is as defined above;
R₇ is α-R₇₋₁:β-R₇₋₂ where R₇₋₁ is -H, -F, -Cl, -Br, -I, -OR₇₋₃ where R₇₋₃ is -H or C₁-C₆ alkyl, and where R₇₋₂ is -H, -CH₃, -F, -Cl, -Br, -I, -OR₇₋₄ where R₇₋₄ is -H or C₁-C₆ alkyl, -SR₇₋₄ where R₇₋₄ is as defined above, with the proviso that one of R₇₋₁ and R₇₋₂ is -H;
(C-I) R₁₁ is α-H:β-O-, where β-O- is taken together with R₉ to form an epoxide between C₉ and C₁₁;
(C-II) R₉ is -H, -F or -Cl and R₁₁ is =O or α-H:β-R₁₁₋₁ where R₁₁₋₁ is -H or -OH;
R₁₆ is α-R₁₆₋₁:β-R₁₆₋₂ where one of R₁₆₋₁ and R₁₆₋₂ is -H and the other is -H, -OH and -CH₃ which comprises
(1) contacting a 21-unsaturated progesterone of formula (I) 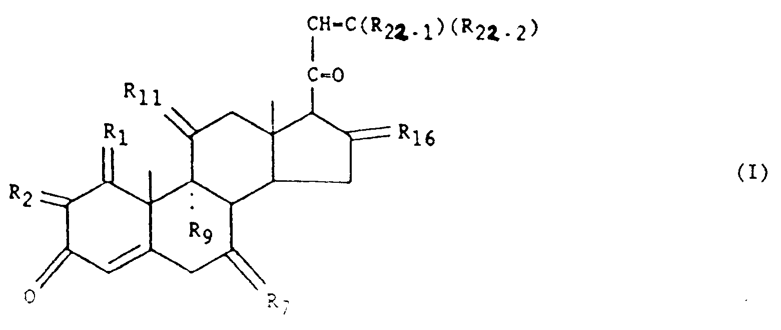 where R₁, R₂, R₇, R₉, R₁₁ and R₁₆ are as defined above, and where R₂₂₋₁ is -H,
-OH,
C₁-C₆ alkyl,
C₄₋C₇ cycloalkyl,
-φ optionally substituted with 1-5 -NO₂, -F, -Cl, -Br, CN, C₁-C₃ alkyl, -OR₂₂₋₃ where R₂₂₋₃ is C₁₋C₃ alkyl, -NR₂₂₋₄R₂₂₋₅ where R₂₂₋₄ and R₂₂₋₅ are the same or different and are selected from the group consisting of -H, C₁₋C₄ alkyl and C₄-C₇ cycloalkyl,
-O-R₂₂₋₆ where R₂₂₋₆ is C₁-C₆ alkyl, C₄₋C₇ cycloalkyl and -φ; R₂₂₋₂ is -H,
C₁-C₆ alkyl,
C₄-C₇ cycloalkyl,
-φ optionally substituted with 1-5 -NO₂, -F, -Cl, -Br, -CN, C₁-C₃ alkyl, -OR₂₂₋₇ where R₂₂₋₇ is C₁-C₃ alkyl, -NR₂₂₋₈R₂₂₋₉ where R₂₂₋₈ and R₂₂₋₉ are the same or different and are selected from the group consisting of -H, C₁₋C₄ alkyl and C₄-C₇ cycloalkyl,
-O-R₂₂₋₁₀ where R₂₂₋₁₀ is C₁₋C₆ alkyl, C₄-C₇ cycloalkyl and -φ,
-CO-O-R₂₂₋₁₁ where R₂₂₋₁₁ is C₁-C₁₀ alkyl, C₄-C₇ cycloalkyl or -φ optionally substituted with 1 thru 5 -NO₂, -F, -Cl, -Br, -CN, C₁-C₃ alkyl, -OR₂₂₋₁₂ where R₂₂₋₁₂ is C₁-C₃ alkyl, -NR₂₂₋₁₃R₂₂₋₁₄ where R₂₂₋₁₃ and R₂₂₋₁₄ are the same or different and are selected from H and C₁-C₄ alkyl, with an oxidising agent.

2. A process according to claim 1, where the oxidising agent is RuO₄, persulfate, pervanadate, pertungstate or MnO₄⁻¹.

3. A process according to claim 1, where the oxidising agent is NaOCl, NaClO₄, IO₄⁻¹ or Ca(OCl)₂.

4. A process according to claim 1, where the oxidising agent is OsO₄/Pb(OAc)₄, KMnO₄/Pb(OAc)₄, OsO₄/IO₄⁻¹.

5. A process according to claim 1, where the oxidising agent is O₃.

6. A process according to claim 5, where at least 2 equivalents of O₃ are used.

7. A process according to any preceding claim, where aqueous hydroxide or a means for generating hydroxide is added following the contacting with the oxidising agent.

8. A process according to claim 7, where the aqueous hydroxide is added in two steps, first water followed by the hydroxide.

9. A process according to claim 7 or claim 8, where the means for generating hydroxide comprises bicarbonate, carbonate or an amine.

10. A process according to any of claims 7 to 9, where the reaction mixture is warmed before or after the addition of the aqueous hydroxide.

11. A process according to any of claims 7 to 10, where the aqueous hydroxide is neutralised by acid such as hydrochloric, sulfuric, phosphoric, nitric, acetic, perchloric, trifluoroacetic, citric, succinic, maleic, benzoic or p-toluenesulfonic acid.

12. A process according to any of claims 7 to 11 when appendent to claim 5 or claim 6, where any excess O₃ is removed prior to the addition of the aqueous hydroxide.

13. A process according to claim 12, where H₂O₂ is added following removal of the excess O₃ and prior to the addition of the aqueous hydroxide.

14. A process according to any preceding claim, where at least 4 equivalents of the oxidising agent are used.

15. A process according to any preceding claim, where R₂₂₋₁ is -OH and R₂₂₋₂ is (C₁-C₄ alkoxycarbonyl).

16. A 3,5-secoandrostan-5-one-3,17β-dioic acid of formula (II-C) 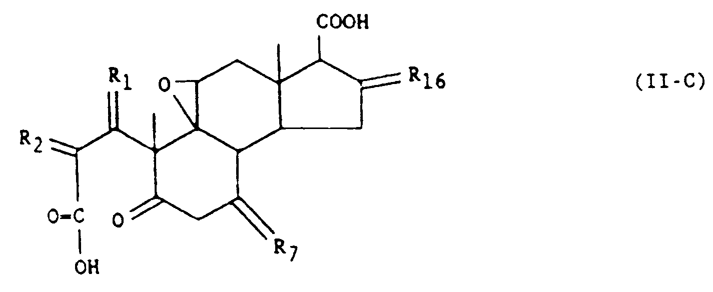 where R₁, R₂, R₇ and R₁₆ are as defined in claim 1.

17. A 21-unsaturated progesterone of formula (I-C) 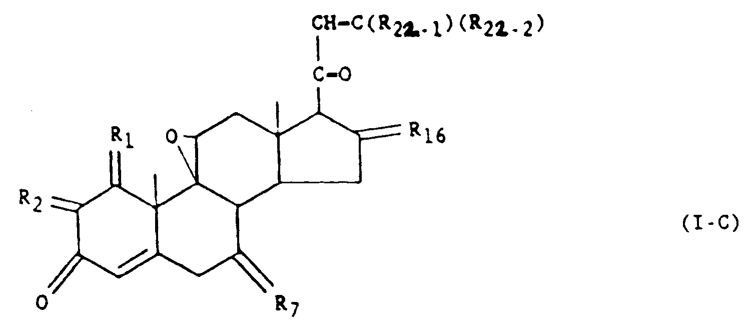 where R₁, R₂, R₇, R₂₂₋₁ and R₂₂₋₂ are as defined in claim 1.

18. A 21-unsaturated progesterone (IC) according to claim 17, where R₂₂₋₁ and R₂₂₋₂ are as defined in claim 15.

## Patentansprüche

1. Verfahren zur Herstellung einer 3,5-Secoandrostan-5-on-3,17β-disäure der Formel (II) 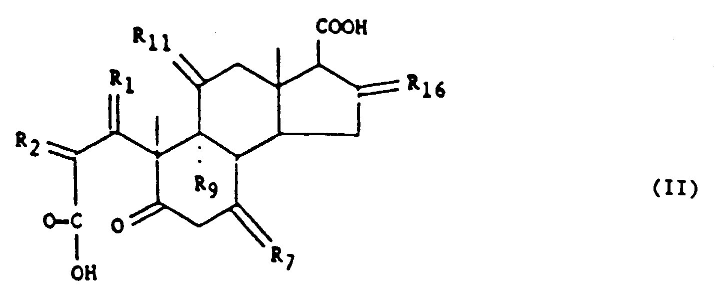 worin bedeuten:
(A-I) R₁ α-R₁₋₁:β-R₁₋₂, wobei einer der Reste R₁₋₁ und R₁₋₂ für -H steht und der andere der Reste R₁₋₁ und R₁₋₂ -H, -F, -Cl, -Br, -I, -OR₁₋₃ mit R₁₋₃ gleich -H oder C₁-C₆ Alkyl, -SR₁₋₃ mit R₁₋₃ in der zuvor angegebenen Bedeutung darstellt, R₂ -H:-H;
(A-II) R₁ -H:-H, R₂ α-R₂₋₁:β-R₂₋₂, wobei einer der Reste R₂₋₁ und R₂₋₂ für -H steht und der andere der Reste R₂₋₁ und R₂₋₂ -H, -F, -Cl, -Br, -I, -OR₂₋₃ mit R₂₋₃ gleich -H oder C₁-C₆ Alkyl, -SR₂₋₃ mit R₂₋₃ in der zuvor angegebenen Bedeutung darstellt;
R₇ α-R₇₋₁:β-R₇₋₂ mit R₇₋₁ gleich -H, -F, -Cl, -Br, -I, -OR₇₋₃ mit R₇₋₃ gleich -H oder C₁-C₆ Alkyl und R₇₋₂ gleich -H, -CH₃, -F, -Cl, -Br, -I, -OR₇₋₄ mit R₇₋₄ gleich -H oder C₁-C₆ Alkyl, -SR₇₋₄ mit R₇₋₄ in der zuvor angegebenen Bedeutung, wobei gilt, daß einer der Reste R₇₋₁ und R₇₋₂ für -H steht;
(C-I) R₁₁ α-H:β-O-, wobei β-O- zusammen mit R₉ zwischen C₉ und C₁₁ ein Epoxid bildet;
(C-II) R₉ -H, -F oder -Cl und R₁₁ =O oder α-H:β-R₁₁₋₁ mit R₁₁₋₁ gleich -H oder -OH;
R₁₆ α-R₁₆₋₁:β-R₁₆₋₂, wobei einer der Reste R₁₆₋₁ und R₁₆₋₂ für -H steht und der andere -H, -OH oder -CH₃ bedeutet, durch
(1) Inberührungbringen eines 21-ungesättigten Progesterons der Formel (I) 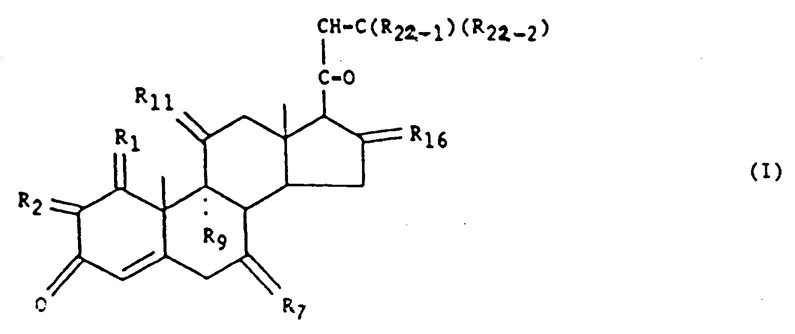 worin R₁, R₂, R₇, R₉, R₁₁ und R₁₆ die zuvor angegebene Bedeutung besitzen und worin R₂₂₋₁ für -H,
-OH,
C₁-C₆ Alkyl,
C₄-C₇ Cycloalkyl,
-φ, gegebenenfalls substituiert durch 1-5 -NO₂, -F, -Cl, -Br, -CN, C₁-C₃ Alkyl, -OR₂₂₋₃ mit R₂₂₋₃ gleich C₁-C₃ Alkyl, -NR₂₂₋₄R₂₂₋₅ mit R₂₂₋₄ und R₂₂₋₅, die gleich oder verschieden sein können, ausgewählt aus der Gruppe -H, C₁-C₄ Alkyl und C₄-C₇ Cycloalkyl,
-O-R₂₂₋₆ mit R₂₂₋₆ gleich C₁-C₆ Alkyl, C₄-C₇ Cycloalkyl und -φ steht und
R₂₂₋₂ -H,
C₁-C₆ Alkyl,
C₄-C₇ Cycloalkyl,
-φ, gegebenenfalls substituiert durch 1-5 -NO₂, -F, -Cl, -Br, -CN, C₁-C₃ Alkyl, -OR₂₂₋₇ mit R₂₂₋₇ gleich C₁-C₃ Alkyl, -NR₂₂₋₈R₂₂₋₉ mit R₂₂₋₈ und R₂₂₋₉, die gleich oder verschieden sein können, ausgewählt aus der Gruppe -H, C₁-C₄ Alkyl und C₄-C₇ Cycloalkyl,
-O-R₂₂₋₁₀ mit R₂₂₋₁₀ gleich C₁-C₆ Alkyl, C₄-C₇ Cycloalkyl oder -φ,
-CO-O-R₂₂₋₁₁ mit R₂₂₋₁₁ gleich C₁-C₁₀ Alkyl, C₄-C₇ Cycloalkyl oder -φ, gegebenenfalls substituiert mit 1 bis 5 -NO₂, -F, -Cl, -Br, -CN, C₁-C₃ Alkyl, -OR₂₂₋₁₂ mit R₂₂₋₁₂ gleich C₁-C₃ Alkyl, -NR₂₂₋₁₃R₂₂₋₁₄ mit R₂₂₋₁₃ und R₂₂₋₁₄, die gleich oder verschieden sein können, ausgewählt aus H und C₁-C₄ Alkyl, darstellt, mit einem Oxidationsmittel.

2. Verfahren nach Anspruch 1, wobei das Oxidationsmittel aus RuO₄, Persulfat, Pervanadat, Perwolframat oder MnO₄⁻¹ besteht.

3. Verfahren nach Anspruch 1, wobei das Oxidationsmittel aus NaOCl, NaClO₄, IO₄⁻¹ oder Ca(OCl)₂ besteht.

4. Verfahren nach Anspruch 1, wobei das Oxidationsmittel aus OsO₄/Pb(OAc)₄, KMnO₄/Pb(OAc)₄ oder OsO₄/IO₄⁻¹ besteht.

5. Verfahren nach Anspruch 1, wobei das Oxidationsmittel aus O₃ besteht.

6. Verfahren nach Anspruch 5, wobei mindestens zwei Äquivalente O₃ benutzt werden.

7. Verfahren nach einem der vorhergehenden Ansprüche, wobei nach dem Inberührungbringen mit dem Oxidationsmittel ein wäßriges Hydroxid oder ein Hydroxid lieferndes Mittel zugegeben wird.

8. Verfahren nach Anspruch 7, wobei das wäßrige Hydroxid in zwei Stufen, zunächst Wasser und dann das Hydroxid, zugegeben wird.

9. Verfahren nach Anspruch 7 oder 8, wobei das Mittel zur Erzeugung von Hydroxid Bicarbonat, Carbonat oder ein Amin umfaßt.

10. Verfahren nach einem der Ansprüche 7 bis 9, wobei das Reaktionsgemisch vor oder nach der Zugabe des wäßrigen Hydroxids erwärmt wird.

11. Verfahren nach einem der Ansprüche 7 bis 10, wobei das wäßrige Hydroxid durch eine Säure, z.B. Chlorwasserstoff-, Schwefel-, Phosphor-, Salpeter-, Essig-, Perchlor-, Trifluoressig-, Zitronen-, Bernstein-, Malein-, Benzoe- oder p-Toluolsulfonsäure, neutralisiert wird.

12. Verfahren nach einem der Ansprüche 7 bis 11 in Abhängigkeit von Ansprüchen 5 oder 6, wobei jegliches überschüssiges O₃ vor der Zugabe des wäßrigen Hydroxids entfernt wird.

13. Verfahren nach Anspruch 12, wobei nach der Entfernung des überschüssigen O₃ und vor der Zugabe des wäßrigen Hydroxids H₂O₂ zugegeben wird.

14. Verfahren nach einem der vorhergehenden Ansprüche, wobei mindestens 4 Äquivalente Oxidationsmittel verwendet werden.

15. Verfahren nach einem der vorhergehenden Ansprüche, wobei R₂₂₋₁ für -OH steht und R₂₂₋₂ (C₁-C₄ Alkoxycarbonyl) bedeutet.

16. Eine 3,5-Secoandrostan-5-on-3,17β-disäure der Formel (II-C) 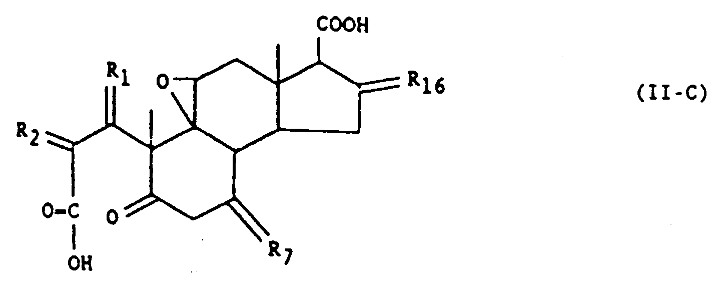 worin R₁, R₂, R₇ und R₁₆ die in Anspruch 1 angegebene Bedeutung besitzen.

17. Ein 21-ungesättigtes Progesteron der Formel (I-C) 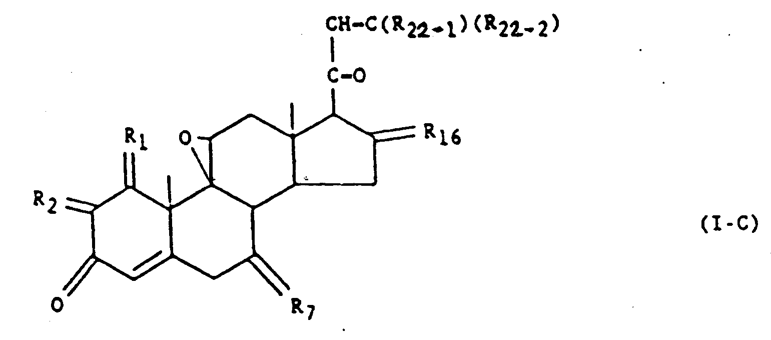 worin R₁, R₂, R₇, R₂₂₋₁ und R₂₂₋₂ die in Anspruch 1 angegebene Bedeutung besitzen.

18. Ein 21-ungesättigtes Progesteron (IC) nach Anspruch 17, wobei R₂₂₋₁ und R₂₂₋₂ die in Anspruch 15 angegebene Bedeutung besitzen.

## Revendications

1. Procédé de production d'un acide 3,5-secoandrostan-5-one-3,17β-dioïque de formule (II) 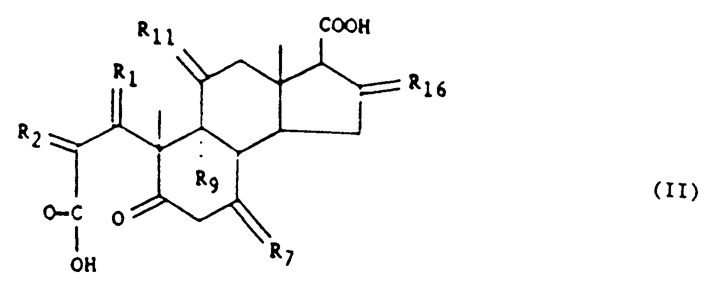 dans laquelle (A-I) R₁ représente un groupe α-R₁₋₁:β-R₁₋₂ dans lequel un des groupes R₁₋₁ et R₁₋₂ représente -H et l'autre des groupes R₁₋₁ et R₁₋₂ représente -H, -F, -Cl, -Br, -I, un groupe -OR₁₋₃ dans lequel R₁₋₃ représente -H ou un groupe alkyle en C₁ à C₆, un groupe -SR₁₋₃ dans lequel R₁₋₃ répond à la définition précitée, R₂ représente -H:-H ;
(A-II) R₁ représente un groupe -H:-H, R₂ représente un groupe α-R₂₋₁:β-R₂₋₂ dans lequel un des groupes R₂₋₁ et R₂₋₂ représente -H et l'autre des groupes R₂₋₁ et R₂₋₂ représente -H, -F, -Cl, -Br, -I, un groupe -OR₂₋₃ dans lequel R₂₋₃ représente -H ou un groupe alkyle en C₁ à C₆, un groupe -SR₂₋₃ dans lequel R₂₋₃ répond à la définition précitée ;
R₇ représente un groupe α-R₇₋₁:β-R₇₋₂ dans lequel R₇₋₁ représente -H, -F, -Cl, -Br, -I, un groupe -OR₇₋₃ dans lequel R₇₋₃ représente -H ou un groupe alkyle en C₁ à C₆, et dans lequel R₇₋₂ représente -H, un groupe -CH₃, -F, -Cl, -Br, -I, un groupe -OR₇₋₄ dans lequel R₇₋₄ représente -H ou un groupe alkyle en C₁ à C₆, un groupe -SR₇₋₄ dans lequel R₇₋₄ répond à la définition précitée, sous réserve qu'un des groupes R₇₋₁ et R₇₋₂ représente -H ;
(C-I) R₁₁ représente α-H:β-O-, dans lequel β-O- est pris conjointement avec R₉ pour former un époxyde entre C₉ et C₁₁ ;
(C-II) R₉ représente -H, -F ou -Cl et R₁₁ représente -O ou un groupe α-H:β-R₁₁₋₁ dans lequel R₁₁₋₁ représente -H ou un groupe -OH ;
R₁₆ représente un groupe α-R₁₆₋₁:β-R₁₆₋₂ dans lequel un des groupes R₁₆₋₁ et R₁₆₋₂ représente -H et l'autre représente -H, -OH ou CH₃, qui comprend
(1) la mise en contact d'une progestérone insaturée en position 21, de formule (I) 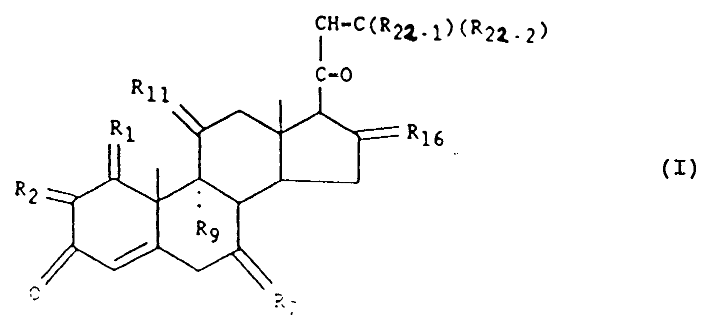 dans laquelle R₁, R₂, R₇, R₉, R₁₁ et R₁₆ répondent aux définitions précitées, et dans laquelle R₂₂₋₁ représente -H,
un groupe -OH,
un groupe alkyle en C₁ à C₆,
un groupe cycloalkyle en C₄ à C₇,
un groupe -φ facultativement substitué avec 1 à 5 groupes -NO₂, -F, -Cl, -Br, -CN, alkyle en C₁ à C₃, -OR₂₂₋₃ dans lequel R₂₂₋₃ représente un groupe alkyle en C₁ à C₃, -NR₂₂₋₄R₂₂₋₅ sont identiques ou différents et sont choisis dans le groupe consistant en -H, un groupe alkyle en C₁ à C₄ et un groupe cycloalkyle en C₄ à C₇,
un groupe -OR₂₂₋₆ dans lequel R₂₂₋₆ représente un groupe alkyle en C₁ à C₆, cycloalkyle en C₄ à C₇ ou -φ ;
R₂₂₋₂ représente -H,
un groupe alkyle en C₁ à C₆,
un groupe cycloalkyle en C₄ à C₇,
un groupe -φ facultativement substitué avec 1 à 5 groupes -NO₂, -F, -Cl, -Br, -CN, alkyle en C₁ à C₃, -OR₂₂₋₇ dans lequel R₂₂₋₇ représente groupe alkyle en C₁ à C₃, -NR₂₂₋₈R₂₂₋₉ dans lequel R₂₂₋₈ et R₂₂₋₉ sont identiques ou différents et sont choisis dans le groupe consistant en -H, un groupe alkyle en C₁ à C₄ et un groupe cycloalkyle en C₄ à C₇,
un groupe -O-R₂₂₋₁₀ dans lequel R₂₂₋₁₀ représente un groupe alkyle en C₁ à C₆, cycloalkyle en C₄ à C₇ ou -φ,
un groupe -CO-O-R₂₂₋₁₁ dans lequel R₂₂₋₁₁ représente un groupe alkyle en C₁ à C₁₀, cycloalkyle en C₄ à C₇ ou -φ facultativement substitué avec 1 à 5 groupes -NO₂, -F, -Cl, -Br, -CN, alkyle en C₁ à C₃, -OR₂₂₋₁₂ dans lequel R₂₂₋₁₂ représente un groupe alkyle en C₁ à c₃, -NR₂₂₋₁₃R₂₂₋₁₄ dans lequel R₂₂₋₁₃ et R₂₂₋₁₄ sont identiques ou différents et sont choisis entre -H et un groupe alkyle en C₁ à C₄, avec un agent oxydant.

2. Procédé suivant la revendication 1, dans lequel l'agent oxydant est RuO₄, un persulfate, un pervanadate, un pertungstate ou MnO₄⁻¹.

3. Procédé suivant la revendication 1, dans lequel l'agent oxydant est NaOCl, NaClO₄, IO₄⁻¹ ou Ca(OCl)₂.

4. Procédé suivant la revendication 1, dans lequel l'agent oxydant est OsO₄/Pb(OAc)₄, KMnO₄/Pb(OAc)₄, OsO₄/IO₄⁻¹.

5. Procédé suivant la revendication 1, dans lequel l'agent oxydant est O₃.

6. Procédé suivant la revendication 5, dans lequel au moins deux équivalents de O₃ sont utilisés.

7. Procédé suivant l'une quelconque des revendications précédentes, dans lequel une solution aqueuse d'un hydroxyde ou un moyen pour engendrer un hydroxyde est ajouté après la mise en contact avec l'agent oxydant.

8. Procédé suivant la revendication 7, dans lequel la solution aqueuse d'hydroxyde est ajoutée en deux étapes, l'eau étant ajoutée en premier et étant suivie par l'hydroxyde.

9. Procédé suivant la revendication 7 ou la revendication 8, dans lequel le moyen pour engendrer l'hydroxyde consiste en un bicarbonate, un carbonate ou une amine.

10. Procédé suivant l'une quelconque des revendications 7 à 9, dans lequel le mélange réactionnel est chauffé avant ou après addition de la solution aqueuse d'hydroxyde.

11. Procédé suivant l'une quelconque des revendications 7 à 10, dans lequel la solution aqueuse d'hydroxyde est neutralisée avec un acide tel que l'acide chlorhydrique, sulfurique, phosphorique, nitrique, acétique, perchlorique, trifluoracétique, citrique, succinique, maléique, benzoïque ou p-toluènesulfonique.

12. Procédé suivant l'une quelconque des revendications 7 à 11, lorsqu'elle est annexée à la revendication 5 ou la revendication 6, dans lequel toute quantité de O₃ en excès est éliminée avant addition de la solution aqueuse d'hydroxyde.

13. Procédé suivant la revendication 12, dans lequel H₂O₂ est ajouté après élimination du O₃ en excès et avant l'addition de la solution aqueuse d'hydroxyde.

14. Procédé suivant l'une quelconque des revendications précédentes, dans lequel au moins 4 équivalents de l'agent oxydant sont utilisés.

15. Procédé suivant l'une quelconque des revendications précédentes, dans lequel R₂₂₋₁ représente un groupe -OH et R₂₂₋₂ représente un groupe alkoxycarbonyle en C₁ à C₄.

16. Acide 3,5-secoandrostan-5-one-3,17β-dioïque de formule (II-C) 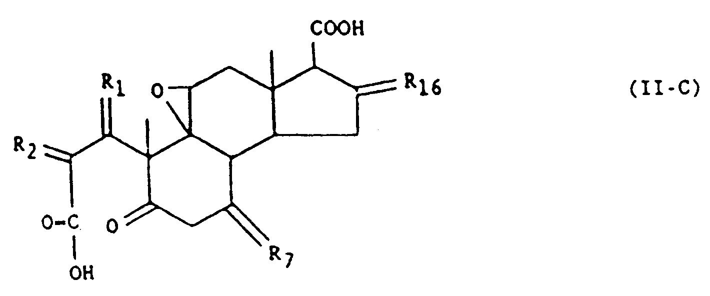 dans laquelle R₁, R₂, R₇ et R₁₆ répondent aux définitions précitées.

17. Progestérone insaturée en position 21, de formule (I-C) 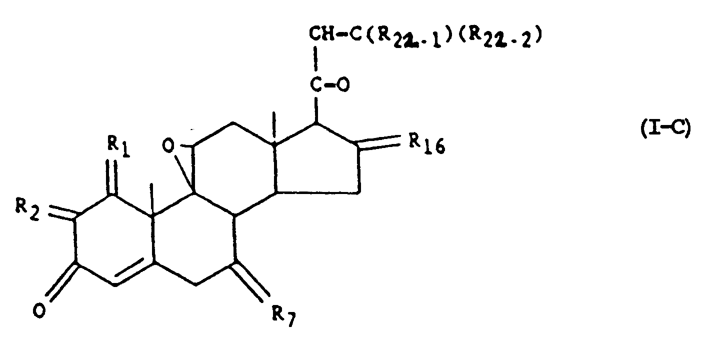 dans laquelle R₁, R₂, R₇ et R₂₂₋₁ et R₂₂₋₂ répondent aux définitions suivant la revendication 1.

18. Progestérone insaturée en position 21, (I-C) suivant la revendication 17, dans laquelle R₂₂₋₁ et R₂₂₋₂ répondent aux définitions suivant la revendication 15.
